# EUROPEAN PATENT APPLICATION

(11) **EP 4 060 930 A2**
(43) Date of publication of application: **21.09.2022**
(21) Application number: 22159742.0
(22) Date of filing: 02.03.2022
(51) Int. Cl.: H04L 7/04, H04L 7/033

(54) **DATA SYNCHRONIZATION DEVICE AND METHOD**

(30) Priority: 19.03.2021 KR 20210036048
(71) Applicant: Todoc Co., Ltd., Guro-gu, Seoul 08394 (KR)
(72) Inventor: PARK, Jong Hyeok, 08394 Seoul (KR); LEE, Ho Seung, 05629 Seoul (KR); KIM, Doo Hee, 04075 Seoul (KR)
(74) Representative: Zimmermann, Tankred Klaus

(57) **Abstract**

Instead of adopting a conventional method of using an average value to generate a clock for reading later data, an embodiment adopts using all count values stored from preamble data. If there are 10 preamble data bits, count values corresponding to the 10 bits are stored and used for a clock for reading later data. A clock count value of the first bit of the preamble is set as a reference count value and + or - values compared to the reference value corresponding to the remaining data bits are stored in a memory as error values of the respective preamble bits. The error values are loaded according to data that is input later and a more accurate synchronization clock is generated. According to the data synchronization device and method, accuracy of data synchronization can be enhanced, and data loss when long-term data synchronization is performed can be prevented.

## Description

### BACKGROUND OF THE INVENTION

### Field of the Invention

The present disclosure relates to a data synchronization method. In particular, the present disclosure relates to a method of synchronizing data when asynchronous data inputs take place.

### Description of the Related Art

Unless otherwise indicated herein, contents set forth in this section are neither the related art to the claims of the present application, nor construed as the related art despite the inclusion in this section.

In general, within one system, a microprocessor that acts as a brain, which is software, and several individual chips are provided. Like human-to-human communication in which the timing of commanding an action and the timing of performing the action differ, the operation time points differ between the processor and the individual chips. This is called an asynchronous system and is a heterogeneous clock domain. In order for the entire system to operate as intended in the heterogeneous clock domain, it is important that when the microprocessor transmits command data to the individual chips, the chips accept the command data according to the synchronization thereof. This is called synchronization.

In the case of a system, such as a cochlear implant, which includes a speech processor as a transmission end, and a nerve stimulator as a reception end, the main processor chip (Master) of the external speech processor transmits data containing information on nerve stimulation and environment settings, to the individual chips (Slave).

Herein, when the power is turned on for data synchronization between the master chip and the slave chips, the processor transmits preamble data one time and the slave chips perform synchronization by using the preamble data. After synchronization is performed, a calculated clock is generated, and the data that will come after the preamble data is loaded according to the calculated clock.

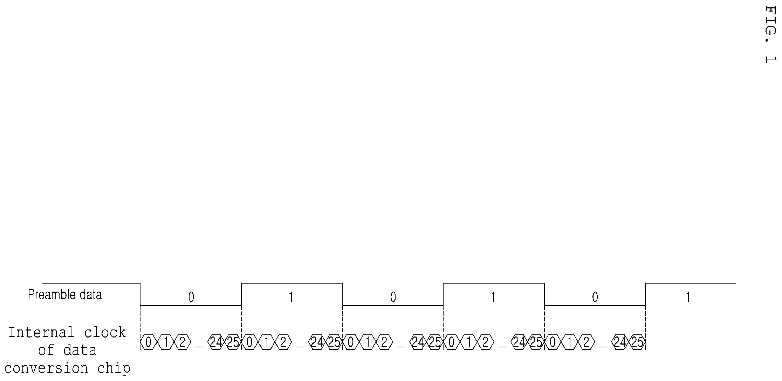

A conventional synchronization method is to count the period of one bit value and store count values during respective bit values, as shown in FIG. 1. In general, an average of stored count values is used to calculate a synchronization clock and the calculated synchronization clock is used in reading the data that will come next.

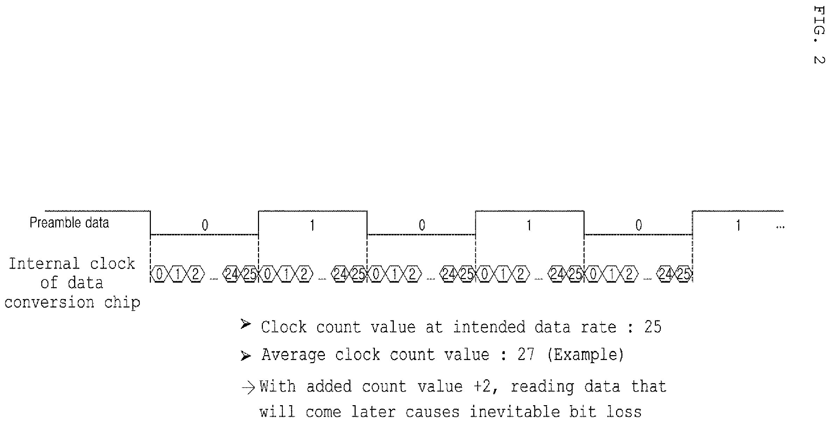

However, in the case of performing synchronization by averaging count values, an intended clock count may be lower or higher than the average clock count value and thus there is a problem that one bit may be lost in the long term. In addition, as shown in FIG. 2, when data is read using an average clock calculated from the slave chips, the time point at which data is read may be greater or less than an intended value because of jitter, and there may be a problem with synchronization and a problem of bit loss due to an increased or decreased accumulated error. Referring to FIG. 2, the clock count value at the intended data rate is 25, but the actual average clock count value is 27. With the count value +2 added to the average clock count value, reading data that will come later causes inevitable bit loss.

The foregoing is intended merely to aid in the understanding of the background of the present disclosure, and is not intended to mean that the present disclosure falls within the purview of the related art that is already known to those skilled in the art.

### Documents of Related Art

(Patent Document 1) Korean Patent No. 10-0986108 (October 01, 2010)
(Patent Document 2) Korean Patent No. 10-1183297 (September 10, 2012)

### SUMMARY OF THE INVENTION

Instead of adopting the conventional method of using an average value to generate a clock for reading later data, an embodiment adopts using all count values stored from preamble data. For example, if there are a total of 10 preamble data bits, count values corresponding to the 10 bits are stored and used for a clock for reading later data. In the embodiment, the clock count value of the first bit of the preamble is set as a reference count value and + or - values compared to the reference value corresponding to the remaining data bits are stored in a memory as error values of the respective preamble bits. Afterward, in the embodiment, the error values stored in the memory are loaded according to data that is input later and a more accurate synchronization clock is generated.

In addition, in the embodiment, in order to prevent data loss from occurring when long-term data synchronization is performed, data, such as preamble data, for synchronization is added in the middle to reduce the probability of loss significantly.

According to an embodiment, there is provided a data synchronization method of a data synchronization device, the method including: (A) storing, by the data synchronization device, count values of respective bits of a preamble; (B) setting, as a reference count value, the number of clocks at a time point at which the first bit of the preamble changes; (C) comparing the set reference count value with the count values of the respective bits included in the preamble; (D) storing, in a memory, increase or decrease values, compared to the reference count value, which are a result of comparison as error values of the respective bits included in the preamble; and (E) loading the error values of the respective bits stored in the memory and generating a synchronization clock.

According to another embodiment, there is provided a data synchronization device including: a reference count setting module configured to store count values of respective bits of a preamble bit and set, as a reference count value, the number of clocks at a time point at which the first bit of the preamble changes; a comparison module configured to compare the set reference count value with the count values of the respective bits included in the preamble, and store, in a memory, increase or decrease values, compared to the reference count value, which are a result of comparison as error values of the respective bits included in the preamble; and a generation module configured to load the error values of the respective bits stored in the memory and generate a synchronization clock.

According to the data synchronization device and method, accuracy of data synchronization can be enhanced, and the data loss when long-term data synchronization is performed can be prevented.

It should be understood that the effects are not limited to those described above, but include all effects that can be inferred from the configurations of the disclosure in the detailed description of the present disclosure or the claims.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a diagram illustrating a data period of a bit value;
FIG. 2 is a diagram illustrating a problem of bit loss caused by a conventional synchronization method;
FIG. 3 is a diagram illustrating data processing blocks of a data synchronization device according to an embodiment;
FIG. 4 is a diagram illustrating an operation process of a data synchronization device according to an embodiment; and
FIG. 5 is a flowchart illustrating data processing of a data synchronization method according to an embodiment.

### DETAILED DESCRIPTION OF THE INVENTION

Advantages and features of the present disclosure, and methods to achieve them will be apparent from the following embodiments that will be described in detail with reference to the accompanying drawings. It should be understood that the present disclosure is not limited to the following embodiments and may be embodied in different ways, and that the embodiments are provided to only complete the disclosure and to allow those skilled in the art to fully understand the category of the disclosure. The disclosure is defined by the category of the claim. Throughout the specification, the same reference numerals refer to the same elements.

In describing the embodiments of the present disclosure, when if it is decided that a detailed description of a well-known function or configuration makes the gist of the present disclosure unclear, the detailed description will be omitted. Further, the terms described below are defined in consideration of the functions in the embodiments of the present disclosure, and may vary depending on the intention of the user, the operator, or the custom. Therefore, the definition should be based on the contents throughout this specification.

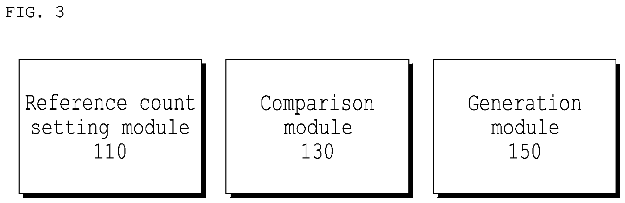

FIG. 3 is a diagram illustrating data processing blocks of a data synchronization device according to an embodiment.

Referring to FIG. 3, the data synchronization device according to the embodiment may include a reference count setting module 110, a comparison module 130, and a generation module 150. The term "module" used herein should be interpreted to include software, hardware, or a combination thereof, depending on the context in which the term is used. For example, software may be machine language, firmware, embedded code, and application software. As another example, hardware may be a circuit, a processor, a computer, an integrated circuit, an integrated circuit core, a sensor, a micro-electro-mechanical system (MEMS), a passive device, or a combination thereof.

The reference count setting module 110 stores count values of respective preamble bits and sets, as a reference count value, the number of clocks at the time point at which the first bit of the preamble changes. The comparison module 130 compares the set reference count value with the count values of the respective bits included in the preamble, and stores increase or decrease values, compared to the reference count value, which are a result of comparison, in a memory as error values of the respective bits included in the preamble. The generation module 150 loads the error values of the respective bits stored in the memory and generates a synchronization clock. That is, the generation module 150 loads the error values stored in the memory according to data that is input later and generates a more accurate synchronization clock.

In an embodiment, when the number of times that the generating of the synchronization clock is performed exceeds a predetermined number or the number of times that synchronization is performed exceeds a predetermined number, resynchronization data including preamble data is added and the reference count setting module 110 perform synchronization again. This is to prevent the data loss when long-term data synchronization is performed. In the embodiment, data, such as preamble data, for synchronization is added during data processing, such as clock counting, so that the probability of data loss can be significantly reduced.

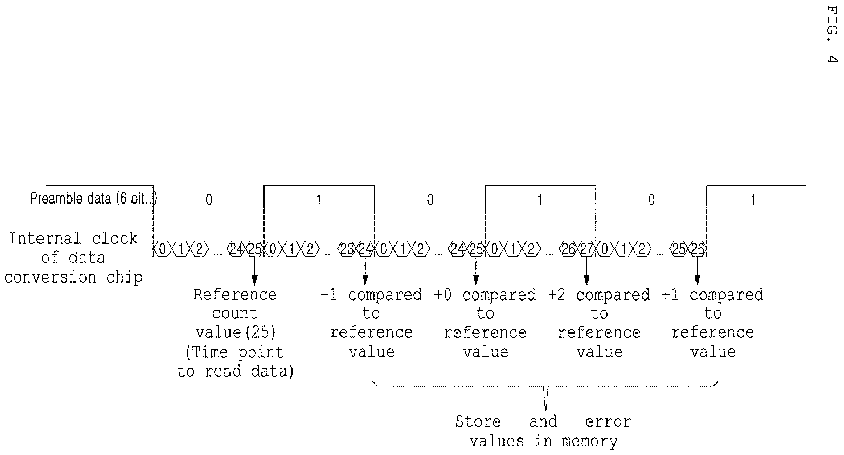

FIG. 4 is a diagram illustrating an operation process of a data synchronization device according to an embodiment.

Referring to FIG. 4, instead of adopting the conventional method of using an average value to generate a clock for reading later data, an embodiment adopts using all count values stored from preamble data. For example, if there are a total of 10 preamble data bits, count values corresponding to the 10 bits are stored and used for a clock for reading later data. Specifically, as shown in FIG. 4, the clock count value of the first bit of the preamble is set as a reference count value. Next, + or - values compared to the reference value corresponding to the remaining data bits are stored in the memory as error values of the respective bits of the preamble. The error values stored in the memory are loaded according to data that is input later and used in generating a more accurate synchronization clock.

Referring to FIG. 4, in the embodiment, the preamble data has 6 bits including values of 0, 1, 0, 1, 0, and 1, and an internal clock of a data conversion chip corresponding to the first bit having a value of 0 in the preamble has a value of 25. In the embodiment, 25, the number of clocks of the first bit of the preamble, is set as a reference count and the internal clocks of the data conversion chip of the subsequent bits are counted. In the embodiment, the second bit having a value of 1 in the preamble has a count value of 24, the third bit having a value of 0 has a count value of 25, the fourth bit having a value of 1 has a count value of 27, and the fifth bit having a value of 0 has a count value of 26. Comparing the count values of the respective bits included in the preamble with the reference count value of 25, the increase and decrease values corresponding to the bits are -1, 0, +2, and +1 in that order. In the embodiment, the error values, -1, 0, +2, and +1, which are the increase and decrease values of the bits are stored in the memory, and the stored error value data is loaded to generate a more accurate synchronization clock.

In addition, in the embodiment, in order to prevent data loss from occurring when long-term data synchronization is performed, for example, the number of times that the generating of the synchronization clock is performed exceeds a predetermined number or the number of times that synchronization is performed exceeds a predetermined number, data, such as preamble data, for synchronization is added in the middle so that the probability of loss can be significantly reduced.

Hereinafter, a data synchronization system to which the data synchronization device according to the present disclosure is applied will be described. However, contents the same as the above-described contents will be omitted.

The data synchronization system according to the present disclosure includes a master chip and a plurality of slave chips. According to the present disclosure, the master chip generates preamble data for performing synchronization. The master chip transmits the generated preamble data to each of the plurality of slave chips. Accordingly, the plurality of slave chips perform synchronization according to the preamble data.

The plurality of slave chips receive preamble data from the master chip. The plurality of slave chips perform synchronization on the basis of the preamble data and generate a synchronization clock for receiving data transmitted from the master chip.

According to the present disclosure, each of the plurality of slave chips includes the reference count setting module, the comparison module, and the generation module as described above.

The reference count setting module stores the count values of the respective bits included in the preamble data, and sets, as a reference count value, the number of clocks at the time point at which the first bit preamble data changes.

The comparison module compares the set reference count value with the count values of the respective bits included in the preamble data, and stores increase or decrease values, compared to the reference count value, which are a result of comparison, in the memory as error values of the respective bits included in the preamble data.

The generation module loads the error values of the respective bits stored in the memory and generates the synchronization clock.

Herein, detailed descriptions of the reference count setting module, the comparison module, and the generation module are the same as those described above.

According to an embodiment of the present disclosure, when synchronization of each slave chips is performed, the master chip transmits data to the plurality of slave chips. When the number of data transmission bits is equal to or greater than a predetermined number of bits, the master chip transmits first preamble data for performing resynchronization. For example, the master chip may transmit the first preamble data on a per 100 bits basis or a per 200 bits basis.

Accordingly, the slave chips perform resynchronization with the first preamble data.

In an embodiment, the slave chips receive different preamble data from the master chip, and perform synchronization on the basis of the different preamble data. Herein, the preamble data that the slave chips receive mean preamble data having different count values.

For example, in the preamble data that a first slave chip receives, the count value of the first bit of the preamble data may be 16, and in the preamble data that a second slave chip receives, the count value of the first bit of the preamble data may be 24.

In this case, the plurality of slave chips set different reference count value of the respective slave chips and thus calculate different increase or decrease values compared to the reference count values. The plurality of slave chips store the different increase or decrease values in the memory as error values of the respective bits included in the preamble data. The stored error values are different between the slave chips and different synchronization clocks are thus generated.

Accordingly, each of the plurality of slave chips generates an optimal synchronization clock.

Hereinafter, a data synchronization method will be described in order. The operation (function) of the data synchronization method according to the embodiment is fundamentally the same as the function of the data synchronization device, so a description the same as that of FIGS. 3 and 4 will be omitted.

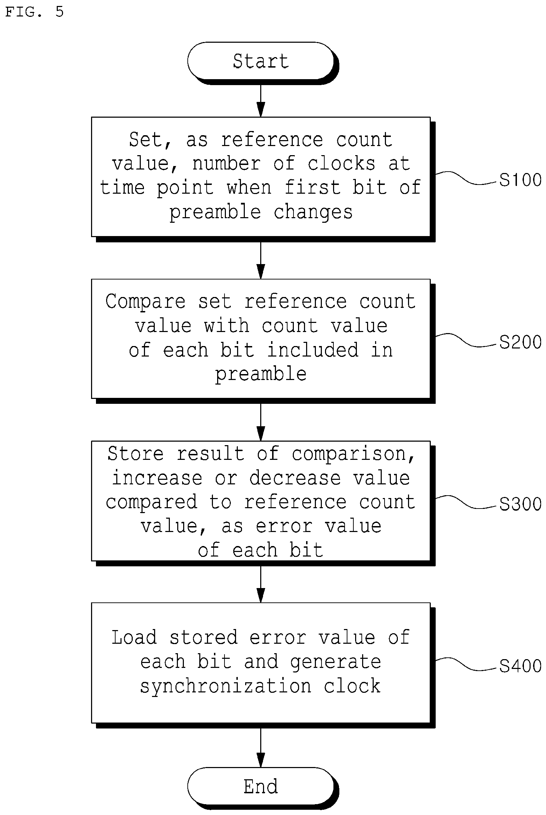

FIG. 5 is a flowchart illustrating data processing of a data synchronization method according to an embodiment.

At step S100, the data synchronization device sets, as a reference count value, the number of clocks at the time point at which the first bit of the preamble among the bits included in the preamble changes. At step S200, the set reference count value is compared with the count values of the respective bits included in the preamble. At step S300, increase or decrease values, compared to the reference count value, which are a result of comparison, are stored in the memory as error values of the respective bits included in the preamble. At step S400, the error values of the respective bits stored in the memory are loaded and a synchronization clock is generated.

In the embodiment, at step S100, resynchronization data including preamble data is added and synchronization is performed again when long-term data synchronization is performed, for example, when the number of times that the generating of the synchronization clock is performed exceeds a predetermined number or the number of times that synchronization is performed exceeds a predetermined number. This is to reduce the probability of data loss. In the embodiment, in order to prevent data loss from occurring when long-term data synchronization is performed, data, such as preamble data, for synchronization is added in the middle.

Instead of adopting the conventional method of using an average value to generate a clock for reading later data, an embodiment adopts using all count values stored from preamble data. For example, if there are a total of 10 preamble data bits, count values corresponding to the 10 bits are stored and used for a clock for reading later data. In the embodiment, the clock count value of the first bit of the preamble is set as a reference count value and + or - values compared to the reference value corresponding to the remaining data bits are stored in a memory as error values of the respective preamble bits. Afterward, in the embodiment, the error values stored in the memory are loaded according to data that is input later and a more accurate synchronization clock is generated. According to the data synchronization device and method, accuracy of data synchronization can be enhanced, and the data loss when long-term data synchronization is performed can be prevented.

Although preferred embodiments of the present disclosure have been described for illustrative purposes, those skilled in the art will appreciate that various modifications, additions, and substitutions are possible, without departing from the scope and spirit of the disclosure as disclosed in the accompanying claims.

## Claims

1. A data synchronization method of a data synchronization device, the method comprising:
(A) setting, as a reference count value by the data synchronization device, the number of clocks at a time point at which the first bit among bits included in a preamble changes;
(B) comparing the set reference count value with count values of the respective bits included in the preamble;
(C) storing, in a memory, increase or decrease values, compared to the reference count value, which are a result of comparison as error values of the respective bits included in the preamble; and
(D) loading the error values of the respective bits stored in the memory and generating a synchronization clock.

2. The method of claim 1, wherein at the step (A), resynchronization data including preamble data is added so that synchronization is performed again when the number of times that the generating of the synchronization clock is performed exceeds a predetermined number or the number of times that synchronization is performed exceeds a predetermined number.

3. A data synchronization device, comprising:
a reference count setting module configured to set, as a reference count value, the number of clocks at a time point at which the first bit among bits included in a preamble changes;
a comparison module configured to compare the set reference count value with count values of the respective bits included in the preamble, and store, in a memory, increase or decrease values, compared to the reference count value, which are a result of comparison as error values of the respective bits included in the preamble; and
a generation module configured to load the error values of the respective bits stored in the memory and generate a synchronization clock.

4. The device of claim 3, wherein the reference count setting module is configured to add resynchronization data including preamble data so that synchronization is performed again when the number of times that the generating of the synchronization clock is performed exceeds a predetermined number or the number of times that synchronization is performed exceeds a predetermined number.

5. A data synchronization system, comprising:
a master chip configured to generate preamble data for performing synchronization; and
a plurality of slave chips each configured to perform synchronization on the basis of the preamble data and generate a synchronization clock for receiving data transmitted from the master chip,
wherein each of the plurality of slave chips comprises:
a reference count setting module configured to set, as a reference count value, the number of clocks at a time point at which the first bit of the preamble data among bits included in the preamble data changes;
a comparison module configured to compare the set reference count value with count values of the respective bits included in the preamble data, and store, in a memory, increase or decrease values, compared to the reference count value, which are a result of comparison as error values of the respective bits included in the preamble data; and
a generation module configured to load the error values of the respective bits stored in the memory and generate the synchronization clock.

6. The system of claim 5, wherein the master chip is configured to transmit data to the plurality of slave chips when synchronization is performed, and transmit first preamble data for performing resynchronization to the plurality of slave chips when the number of data transmission bits is equal to or greater than a predetermined number of bits, and
the plurality of slave chips are configured to perform resynchronization with the first preamble data.

7. The system of claim 5, wherein the plurality of slave chips are configured to receive the preamble data having the different count values from the master chip, and generate the different synchronization clocks by performing synchronization according to the preamble data received by the respective slave chips.
